# EUROPÄISCHE PATENTSCHRIFT

(11) **EP 1 620 416 B1**
(45) Veröffentlichungstag und Bekanntmachung des Hinweises auf die Patenterteilung: **23.07.2008**
(21) Anmeldenummer: 04727899.9
(22) Anmeldetag: 16.04.2004
(51) Int. Cl.: C07D 301/12

(54) **VERFAHREN ZUR HERSTELLUNG VON PROPYLENOXID**
METHOD FOR PRODUCING PROPYLENE OXIDE
PROCEDE DE PRODUCTION D'OXYDE DE PROPYLENE

(30) Priorität: 16.04.2003 DE 10317519
(43) Veröffentlichungstag der Anmeldung: 01.02.2006
(73) Patentinhaber: BASF SE, 67056 Ludwigshafen (DE)
(72) Erfinder: GÖBBEL, Hans-Georg, 67169 Kallstadt (DE); BASSLER, Peter, 68519 Viernheim (DE); TELES, Joaquim, H., 67166 Otterstadt (DE); RUDOLF, Peter, 68526 Ladenburg (DE)
(74) Vertreter: Isenbruck, Günter
(86) Internationale Anmeldenummer: PCT/EP2004/004104
(87) Internationale Veröffentlichungsnummer: WO 2004/092150

(56) Entgegenhaltungen:
- WO-A-00/07965

## Beschreibung

Die vorliegende Erfindung betrifft ein Verfahren zur Herstellung von Propylenoxid, wobei im Rahmen dieses Verfahrens ein Gemisch, das im wesentlichen Propylenoxid und mindestens ein Lösungsmittel, das bevorzugt Methanol ist, enthält, destillativ aufgearbeitet wird. Im Rahmen dieser Aufarbeitung wird ein Brüden einer Destillationskolonne, der im Wesentlichen Propylenoxid enthält, verdichtet, wodurch ein gasförmiger, unter einem bestimmten Druck stehender Brüden erhalten wird. Gemäß einer bevorzugten Ausführungsform wird die in dem komprimierten Brüden enthaltene Energie zumindest teilweise in das Destillationsverfahren zurückgeführt, wobei beispielsweise mindestens ein Verdampfer der Destillationskolonne betrieben wird. Gemäß weiterer bevorzugter Ausführungsformen umfasst die vorliegende Erfindung weitere Aspekte, die sich hinsichtlich des Energiehaushaltes des Gesamtverfahrens positiv auswirken.

In den zahlreichen Veröffentlichungen, die es auf dem Gebiet der Herstellung von Propylenoxid gibt, existieren nur wenige, die integrierte Verfahren betreffen, bei denen in einem destillativen Schritt die Brüdenenergie sinnvoll in das Verfahren rückgeführt wird. Insbesondere gilt dies daher für Verfahren, bei denen Propylenoxid von Lösungsmittel oder Lösungsmittelspuren destillativ getrennt wird.

Die WO 02/14298 A1 beschreibt ein Verfahren zur kontinuierlichen Herstellung eines Olefinoxids. Im Rahmen eines Verfahrenschrittes wird offenbart, dass die Kondensationswärme, die am Kopf einer Kolonne gewonnen wird, für einige oder alle Destillationsprozesse des Gesamtverfahrens rückgewonnen werden kann. In der in Rede stehenden Kolonne wird dabei ein Gemisch destillativ getrennt, das Lösungsmittel, Sauerstoff und Inertgas enthält. Spezielle Verfahrensführungen sind für die Rückführung der Kondensationswärme nicht angegeben.

Die WO 00/07965 beschreibt ein Verfahren zur Herstellung von Propylenoxid, bei dem aus einem Gemisch über Kopf einer Destillationskolonne ein Gemisch aus Propen, Propylenoxid und Methanol abgetrennt wird, wobei in einem Teilkondensator am Kopf der Kolonne der für die Trennung in der Kolonne benötigte Rücklauf kondensiert wird.

Wird bei der Herstellung von Propylenoxid ausgehend von Propen beispielsweise Methanol als Lösungsmittel eingesetzt, ist dies im Allgemeinen für den Reaktionsteil, d.h. für die Umsetzung des Propens mit einem Hydroperoxid wie beispielsweise Wasserstoffperoxid vorteilhaft, insbesondere, wenn als Katalysator zur Umsetzung ein Titansilikalitkatalysator vom Typ TS-1 eingesetzt wird. Andererseits wird die Reinigung des Propylenoxids durch die Anwesenheit von Methanol erschwert.

Bei Normaldruck oder höheren Drücken, die sich im Wesentlichen zwischen 1 und 5 bar bewegen, ist die destillative Trennung von Propylenoxid und Methanol aufgrund des schleppenden Azeotrops nur unter gleichzeitiger Verwendung einer Destillationskolonnen mit sehr vielen theoretischen Trennstufen und Einstellung eines sehr hohen Rücklaufverhältnisses möglich.

Im Bereich niedrigerer Drücke ist die Trennaufgabe einfacher, wobei sich jedoch der niedrige Druck nachteilig auf die Kondensationstemperatur auswirkt, denn die Kondensationstemperatur, die, je nach Druck, beispielsweise bei Werten von ca. 15 °C liegen kann, ist mit der Bereitstellung einer hohen Kondensationskälteleistung verknüpft. Gerade im großtechnischen Maßstab fallen hier enorme Kosten an.

Eine der Aufgaben, die der vorliegenden Erfindung zugrunde lag, war es daher, ein Verfahren bereitzustellen, das im Vergleich zu den im Stand der Technik beschriebenen Verfahren zur Herstellung von Propylenoxid ein deutlich verbesserte Energiebilanz aufweist.

Demgemäß betrifft die vorliegende Erfindung ein Verfahren zur Herstellung von Propylenoxid, mindestens umfassend die Schritte (iii) und (iv)
(iii) Destillative Abtrennung von Propylenoxid aus einem Gemisch (G1), enthaltend Propylenoxid und mindestens ein Lösungsmittel, unter Verwendung einer Destillationskolonne, wobei ein Sumpfstrom und ein Brüdenstrom, im Wesentlichen enthaltend Propylenoxid, erhalten wird;
(iv) Komprimierung des gemäß (iii) erhaltenen Brüdenstroms mittels mindestens eines Verdichters unter Erhalt eines verdichteten Brüden.

Als das mindestens eine gemäß (iii) im Gemisch (G1) enthaltene Lösungsmittel ist grundsätzliches jedes Lösungsmittel denkbar, das im Rahmen des Herstellungsprozesses von Propylenoxid eingesetzt wird.

Insbesondere bevorzugt sind als Lösungsmittel hierbei etwa
- Wasser;
- Alkohole, bevorzugt Alkohole mit weniger als 6 Kohlenstoffatomen, weiter bevorzugt Methanol, Ethanol, Propanole, Butanole, Pentanole;
- Diole oder Polyole, bevorzugt solche mit weniger als 6 Kohlenstoffatomen;
- Ether wie beispielsweise Diethylether, Tetrahydrofuran, Dioxan, 1,2-Diethoxyethan, 2-Methoxyethanol;
- Ester wie beispielsweise Methylacetat oder Butyrolacton;
- Amide wie beispielsweise Dimethylformamid, Dimethylacetamid, N-Methylpyrrolidon;
- Ketone wie beispielsweise Aceton;
- Nitrile wie beispielsweise Acetonitril;
- oder Gemische aus zwei oder mehr der genannten Verbindungen.

Ganz besonders bevorzugt wird im erfindungsgemäßen Verfahren ein Gemisch (G1) eingesetzt, das neben Propylenoxid als Lösungsmittel Methanol oder Wasser oder Wasser und Methanol enthält. Weiter besonders bevorzugt enthält das Gemisch (G1) Methanol als Lösungsmittel.

Demgemäß betrifft die vorliegende Erfindung ein Verfahren, wie oben beschrieben, das dadurch gekennzeichnet ist, dass das mindestens eine Lösungsmittel Methanol ist.

Der Lösungsmittelgehalt und besonders bevorzugt der Methanolgehalt des gemäß (iii) aus dem Gemisch (G1) abgetrennten Propylenoxids liegt im allgemeinen im Bereich von höchstens 500 ppm, bevorzugt von höchstens 200 ppm, weiter bevorzugt von höchstens 100 ppm, weiter bevorzugt von höchstens 50 ppm, weiter bevorzugt von höchstens 20 ppm, besonders bevorzugt von höchstens 15 ppm und insbesondere bevorzugt von höchstens 10 ppm, jeweils bezogen auf das Gesamtgewicht der abgetrennten Propylenoxidfraktion.

Als Destillationskolonne kann im Wesentlichen jede beliebige Kolonne eingesetzt werden. Insbesondere bevorzugt ist eine Destillationskolonne, die als Packungskolonne ausgestaltet ist, wobei weiter bevorzugt eine Packungskolonne mit geordneten Packungen eingesetzt wird. Eine solche Packungskolonne weist eine hohe Trennleistung pro Meter Packung sowie einen nur sehr geringen Druckverlust auf.

Während die genannten geordneten Packungen im Wesentlichen beliebig ausgestaltet sein können, sind solche Packungen bevorzugt, die eine spezifische Oberfläche im Bereich von 100 bis 750 m²/m³ aufweisen. Es können dabei Blechpackungen beispielsweise der Fa. Montz (Typ B1 100 bis B1 500) oder der Fa. Sulzer ChemTech (Typ Mellapak 125 bis Mellapak 750) oder Gewebepackungen der Fa. Montz (Typ A3 500 bis A3 750) oder der Fa. Sulzer ChemTech (Typ BX oder CY) eingesetzt werden. Die Angabe m²/m³ bezieht sich auf die geometrische Oberfläche des für die Packung eingearbeiteten Materials pro Kubikmeter Packung.

Während die Destillation gemäß (iii) generell bei sämtlichen geeigneten Bedingungen durchgeführt werden kann, sind im Rahmen der vorliegenden Erfindung Ausführungsformen der Destillation gemäß (iü) bevorzugt, bei denen das Gemisch (G1) unter Vakuum aufgetrennt wird. Unter dem Begriff "Vakuumdestillation", wie er im Rahmen der vorliegenden Erfindung verwendet wird, wird jede Destillation verstanden, die bei einem Druck von weniger als 1,013 bar durchgeführt wird.

Daher wird die Destillation gemäß (iii) im allgemeinen bei Drücken durchgeführt, die im Bereich von weniger als 1,013 bar, bevorzugt in einem Bereich von höchstens 1 bar, weiter bevorzugt in einem Bereich von 300 bis 900 mbar, weiter bevorzugt in einem Bereich von 400 bis 800 mbar und besonders bevorzugt in einem Bereich von 450 bis 750 mbar liegen.

Demgemäß betrifft die vorliegende Erfindung auch ein Verfahren, wie oben beschrieben, das dadurch gekennzeichnet ist, dass die zur destillativen Abtrennung gemäß (iii) verwendete Destillationskolonne bei einem Druck im Bereich von 450 bis 750 mbar betrieben wird.

Gemäß Schritt (iv) des erfindungsgemäßen Verfahrens wird der am Kopf der Destillationskolonne erhaltene Brüden, der im Wesentlichen aus Propylenoxid besteht, verdichtet. Diese Verdichtung kann generell gemäß sämtlicher geeigneter Methoden erfolgen. Insbesondere kann der Brüden mechanisch oder thermisch verdichtet werden, wobei die Verdichtung in einem oder mehreren Vorrichtungen erfolgen kann. Demgemäß ist es möglich, den Brüden in mindestens einer Verdichtungsvorrichtung mechanisch zu komprimieren oder den Brüden in mindestens einer Verdichtungsvorrichtung thermisch zu komprimieren oder den Brüden zunächst in mindestens einer Verdichtungsvorrichtung mechanisch zu komprimieren und sodann in mindestens einer Verdichtungsvorrichtung thermisch zu komprimieren oder den Brüden zunächst in mindestens einer Verdichtungsvorrichtung thermisch zu komprimieren und sodann in mindestens einer Verdichtungsvorrichtung mechanisch zu komprimieren.

Als Vorrichtungen zur mechanischen Komprimierung sind etwa Drehkolbenverdichter, Schraubenverdichter, Turboverdichter in axialer oder radialer Bauart, Membranverdichter oder Gebläse zu nennen. Zur erfindungsgemäßen Verdichtung kann eine dieser Vorrichtungen oder eine Kombination aus zwei oder mehr dieser Vorrichtungen eingesetzt werden, wobei jeder der eingesetzten Verdichter einstufig oder mehrstufig ausgeführt sein kann.

Als Vorrichtung zur thermischen Verdichtung ist etwa ein Dampfstrahlverdichter zu nennen, der entweder mit einer starren oder mit einer regelbaren Treibdüse ausgestattet sein kann.

Besonders bevorzugt wird der Brüden im Rahmen der vorliegenden Erfindung mechanische verdichtet, wobei wiederum bevorzugt die Verdichtung in einer einzigen Vorrichtung erfolgt. Wiederum bevorzugt ist dabei der Einsatz eines Turboverdichters, und ganz besonders bevorzugt der Einsatz eines einstufigen Einwellenverdichters.

Demgemäß betrifft die vorliegende Erfindung auch ein Verfahren, wie oben beschrieben, das dadurch gekennzeichnet ist, dass die Verdichtung des Brüden mit einem Turboverdichter erfolgt.

Gemäß einer weiter bevorzugten Ausführungsform des erfindungsgemäßen Verfahren wird der Brüden mittels des bevorzugt eingesetzten mechanischen Verdichters derart komprimiert, dass sich der Brüden nach dem Verlassen des Verdichters unter einem Druck in einem Bereich von im allgemeinen 1,5 bis 5 bar, bevorzugt von 2 bis 4 bar und besonders bevorzugt von 2,5 bis 3,5 bar befindet.

Im Allgemeinen wird der Brüden durch die Komprimierung auf eine Temperatur gebracht, die mindestens 1 °C höher liegt als die Temperatur des im Sumpf der Destillationskolonne verdampfenden Mediums. Bevorzugt wird der Brüden durch die Komprimierung auf eine Temperatur gebracht, die im Bereich von 2 bis 25 °C, weiter bevorzugt in einem Bereich von 5 bis 20 °C und besonders bevorzugt in einem Bereich von 8 bis 20 °C höher liegt als die Temperatur des in der Destillationskolonne verdampfenden Mediums.

Demgemäß betrifft die vorliegende Erfindung auch ein Verfahren, wie oben beschrieben, das dadurch gekennzeichnet ist, dass der Brüden gemäß (iv) auf einen Druck im Bereich von 2 bis 5 bar komprimiert wird und der komprimierte Brüden eine Temperatur aufweist, die in einem Bereich von 8 bis 20 °C über der Temperatur des in der Destillationskolonne gemäß (iii) verdampfenden Mediums liegt.

Durch die erfindungsgemäße Verdichtung ermöglicht es das erfindungsgemäße Verfahren daher, den wie oben beschrieben günstigen Druckbereich der Destillation von unter 1,013 bar, bevorzugt den Bereich von 450 bis 750 mbar, zu verwenden, ohne den Nachteil der niedrigen Kondensationstemperatur und der damit zur Verfügung zu stellenden hohen Kälteleistung in Kauf nehmen zu müssen.

In Abhängigkeit von der Zusammensetzung des Zulaufs und der geforderten Reinheit des Propylenoxids hinsichtlich der Restkonzentration an Lösungsmittel, bevorzugt Methanol, ergibt sich eine Verdichterleistung im Bereich von 3 bis 9 MW. Die entsprechende Kondensations-/Kälteleistung, die aufzubringen wäre, läge bei einer Temperatur im Bereich von 12 bis 20 °C in einem Bereich von 15 bis 25 MW.

Die durch die Komprimierung im Brüden zusätzlich gespeicherte Energie kann beispielsweise bevorzugt in irgendein Verfahren eingespeist werden, wobei die Rückführung in das erfindungsgemäße Verfahren bevorzugt ist. Generell kann die Energiemenge ganz oder teilweise jedem beliebigen Verfahrensschritt zugeführt werden. Besonders bevorzugt ist hierbei weiterhin die Rückführung mindestens eines Teils der im komprimierten Brüden gespeicherten Energie in den destillativen Schritt gemäß (iii). Besonders bevorzugt ist hierbei wiederum, dass durch die im komprimierten Brüden gespeicherte Energie mindestens ein Verdampfer der Destillationskolonne wie beispielsweise mindestens ein Zwischenverdampfer oder der Hauptverdampfer oder mindestens ein Zwischenverdampfer und der Hauptverdampfer betrieben werden. Auf diese Art und Weise wird im erfindungsgemäßen Verfahren durch diese integrierte Verfahrensführung eine Wärmepumpe realisiert.

Gemäß einer ganz besonders bevorzugten Ausführungsform wird der komprimierte gasförmige Brüden in mindestens einem Kondensator verflüssigt und die Kondensationswärme zumindest teilweise zum Betrieb mindestens eines der oben genannten Verdampfer eingesetzt. Besonders bevorzugt ist der Betrieb des Hauptverdampfers der gemäß (iii) eingesetzten Destillationskolonne.

Demgemäß betrifft die vorliegende Erfindung auch ein Verfahren, wie oben beschrieben, das zusätzlich den folgenden Schritt (v) umfasst
(v) Kondensation des gemäß (iv) erhaltenen Brüden unter Rückführung mindestens einen Teils der Kondensationswärme in mindestens einen in der gemäß (iii) verwendeten Destillationskolonne eingesetzten Verdampfer.

Die Kondensation gemäß (v) erfolgt hierbei in einem Verdampfer, der im Wesentlichen beliebig ausgestaltet sein kann. Beispiele für die Ausführungsform des Verdampfers sind etwa Naturumlaufverdampfer, Zwangsumlaufverdampfer oder Fallfilmverdampfer. Bevorzugt wird im Rahmen der vorliegenden Erfindung ein Verdampfer eingesetzt, der als Naturumlaufverdampfer ausgestaltet ist.

Das nach Verlassen des mindestens einen Kondensators gemäß (v) abgekühlte Kondensat weist im erfindungsgemäßen Verfahren eine Temperatur im Bereich von im allgemeinen 40 bis 75 °C, bevorzugt im Bereich von 45 bis 70 °C und besonders bevorzugt im Bereich von 45 bis 65 °C auf.

Generell ist es möglich, zum Betrieb des Verdampfers lediglich die aus der Kondensation des verdichteten Brüden wie oben beschrieben gewonnene Energie einzusetzen. Gemäß einer weiter bevorzugten Ausführungsform des erfindungsgemäßen Verfahrens wird zusätzlich mindestens ein weiterer Verdampfer vorgesehen, der dazu dient, Unterschiede in der Kondensations- und Verdampfungsenthalpie aufzufangen, und der demgemäß als Zusatz- oder Nebenverdampfer fungiert.

Dieser mindestens eine zusätzliche Verdampfer kann im Wesentlichen beliebig ausgestaltet sein kann. Beispiele für die Ausführungsform des mindestens einen zusätzlichen Verdampfers sind etwa Naturumlaufverdampfer, Zwangsumlaufverdampfer oder Fallfilmverdampfer. Bevorzugt wird im Rahmen der vorliegenden Erfindung ein Verdampfer eingesetzt, der als Naturumlaufverdampfer ausgestaltet ist.

Gemäß einer weiter bevorzugten Ausführungsform des erfindungsgemäßen Verfahrens wird mindestens ein Teil des gemäß (v) erhaltenen Kondensats in mindestens einem weiteren Wärmetauscher weiter abgekühlt, womit wiederum Energie gewonnen wird, die irgend einem anderen Verfahren zugeführt oder bevorzugt in das erfindungsgemäße Verfahren rückgeführt werden kann.

Bevorzugt wird dieser Teil des Kondensats in dem mindestens einen weiteren Wärmetauscher auf eine Temperatur im Bereich von 10 bis 30 °C und besonders bevorzugt im Bereich von 12 bis 20 °C abgekühlt.

In einer ganz besonders bevorzugten Ausführungsform wird das nach Verlassen dieses mindestens einen Wärmetauschers abgekühlte Kondensat als Rücklauf auf die gemäß (iii) eingesetzte Destillationskolonne rückgeführt.

Demgemäß betrifft die vorliegende Erfindung auch ein Verfahren, wie oben beschrieben, das zusätzlich folgenden Schritt (vi) umfasst
(vi) Abkühlen mindestens eines Teils des gemäß (v) erhaltenen Kondensats in mindestens einem Wärmetauscher auf eine Temperatur im Bereich von 10 bis 30 °C und Rückführung dieses Teils des abgekühlten Kondensats als Rücklauf auf die gemäß (iii) verwendete Destillationskolonne.

Die in dem Wärmetauscher gemäß (vi) zum Abkühlen des Kondensats aufzubringende Kälteleistung wird im erfindungsgemäßen Verfahren bevorzugt durch mindestens einen Teilprozess des erfindungsgemäßen Verfahrens bereitgestellt. Beispielsweise ist es denkbar, dass in dem Wärmetauscher gemäß (vi) die Kälteleistung einem Kältemittel entzogen wird, das an anderer Stelle des Verfahrens die derart entzogene Kältemenge wieder aufnimmt. Ebenso ist es jedoch denkbar, dass in dem Wärmetauscher die aufgenommene Kälteleistung direkt von einem Stoff oder einem Stoffgemisch, das generell in jedem möglichen Aggregatzustand vorliegen kann, übertragen wird. Beispielsweise bevorzugt wird im erfindungsgemäßen Verfahren ein komprimierter Strom in ein Kompartiment des Wärmetauschers hinein entspannt und zumindest teilweise, bevorzugt vollständig verdampft und die dabei abgegebene Kälteleistung auf das sich in einem anderen Kompartiment des Wärmetauschers befindliche Kondensat übertragen. Wiederum bevorzugt ist hierbei eine Ausführungsform, gemäß der dieser komprimierte Strom ein komprimierter Propenstrom ist. Insbesondere bevorzugt handelt es sich bei diesem Propenstrom um einen komprimierten Propenstrom, der zunächst, wie beschrieben, in den Wärmetauscher hinein entspannt und im Wärmetauscher verdampft wird und der anschließend in dem wie unten beschriebenen Schritt (i) des erfindungsgemäßen Verfahrens eingesetzt wird.

Besonders bevorzugt wird der komprimierte Propenstrom in dem mindestens einen gemäß (vi) eingesetzten Verdampfer vollständig verdampft.

Demgemäß betrifft die vorliegende Erfindung auch ein Verfahren, wie oben beschrieben, das dadurch gekennzeichnet ist, dass in dem mindestens einen gemäß (vi) eingesetzten Wärmetauscher komprimiertes Propen unter Entspannung vollständig verdampft wird.

Beispielsweise bevorzugt ist der Propenstrom bei einer Temperatur im Bereich 5 bis 30 °C, bevorzugt von 10 bis 30 °C, weiter bevorzugt von 15 bis 30 °C und besonders bevorzugt von 20 bis 30 °C auf einen Druck im Bereich von 20 bis 35 bar komprimiert und wird erfindungsgemäß in Schritt (vi) auf einen Druck im Bereich von 4 bis 10 bar, bevorzugt 5 bis 9 bar und weiter bevorzugt 5 bis 8 bar entspannt und durch Zufuhr von Wärme vollständig verdampft. Beispielsweise wird in etwa die Hälfte der Entspannungskälte des Propens durch diesen Schritt aufgebracht.

Die Entspannung des komprimierten Stroms gemäß (vi) erfolgt hierbei in einen Wärmetauscher hinein, der im Wesentlichen beliebig ausgestaltet sein kann. Beispiele für die Ausführungsform des Wärmetauschers sind etwa Rohrbündelwärmetauscher, Spiralwärmetauscher oder Plattenwärmetauscher. Bevorzugt wird im Rahmen der vorliegenden Erfindung ein Wärmetauscher eingesetzt, der als Rohrbündelwärmetauscher ausgestaltet ist.

Der gemäß (iii) erhaltene Sumpfstrom kann gemäß einer weiter bevorzugten Ausführungsform des erfindungsgemäßen Verfahrens ebenfalls dazu verwendet werden, das erfindungsgemäße Verfahren energetisch noch integrierter zu gestalten.

Diesbezüglich bevorzugt wird die in dem gemäß (iii) erhaltenen Sumpfstrom enthaltene Wärmemenge zumindest teilweise eingesetzt, um das Gemisch (G1) aufzuheizen, bevor es in die Destillationskolonne gemäß (iii) eingebracht wird. Insbesondere bevorzugt wird hierbei ein Wärmetauscher eingesetzt, der als Gegenstromwärmetauscher (Plattenwärmetauscher) ausgestaltet ist.

Demgemäß betrifft die vorliegende Erfindung auch ein Verfahren, wie oben beschrieben, das dadurch gekennzeichnet ist, dass die im gemäß (iii) erhaltenen Sumpfstrom gespeicherte Energie zumindest teilweise zur Aufheizung des Gemisches (G1) vor dessen destillativer Auftrennung gemäß (iii) eingesetzt wird.

Das gemäß (iii) eingesetzte Gemisch (G1) resultiert aus im Wesentlichen beliebigen Verfahrensschritten bei der Herstellung von Propylenoxid unter der Maßgabe, dass ein wie oben beschriebenes Gemisch (G1) erhalten wird.

Besonders bevorzugt wird das Gemisch (G1) aus einem Verfahren erhalten, in dem Propen mit einem Hydroperoxid in Methanol als Lösungsmittel in Anwesenheit eines Zeolith-Katalysator umgesetzt wird.

Im erfindungsgemäßen Verfahren wird zur Umsetzung des Propens mindestens ein Hydroperoxid eingesetzt. Im Rahmen der vorliegenden Anmeldung wird unter dem Begriff "Hydroperoxid" eine Verbindung der allgemeinen Formel ROOH verstanden. Details bezüglich der Hydroperoxidherstellung und von unter anderem im Rahmen des erfindungsgemäßen Verfahrens einsetzbaren Hydroperoxiden sind in der DE-A 198 35 907 zu entnehmen, deren diesbezüglicher Inhalt in den Kontext der vorliegenden Anmeldung aufgenommen wird. Beispiele für erfindungsgemäß einsetzbare Hydroperoxide sind unter anderem tert-Butylhydroperoxid, Ethylbenzolhydroperoxid, tert-Amylhydroperoxid, Cumenhydroperoxid, Cyclohexylhydroperoxid, Methylcyclohexylhydroperoxid, Tetrahydronaphthalinhydroperoxid, Isobutylbenzolhydroperoxid, Ethylnaphthalinhydroperoxid, Persäuren wie beispielsweise Peressigsäure oder Wasserstoffperoxid. Auch Gemische aus zwei oder mehr Hydroperoxiden sind erfindungsgemäß einsetzbar. Bevorzugt wird im Rahmen der vorliegenden Erfindung Wasserstoffperoxid als Hydroperoxid eingesetzt, wobei weiter bevorzugt eine wässrige Wasserstoffperoxidlösung eingesetzt wird.

Bezüglich der im Rahmen der vorliegenden Erfindung einsetzbaren Zeolith-Katalysatoren existieren keine besonderen Beschränkungen.

Zeolithe sind bekanntermaßen kristalline Alumosilikate mit geordneten Kanal- und Käfigstrukturen, die Mikroporen aufweisen, die vorzugsweise kleiner als ungefähr 0,9 nm sind. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W. M. Meier, D. H. Olson und Ch. Baerlocher, "Atlas of Zeolithe Structure Types", Elsevier, 5. Auflage, Amsterdam 2001.

Es sind nun auch Zeolithe bekannt, die kein Aluminium enthalten und bei denen im Silikatgitter an Stelle des Si(IV) teilweise Titan als Ti(IV) steht. Diese Titanzeolithe, insbesondere solche mit einer Kristallstruktur von MFI-Typ, sowie Möglichkeiten zu Ihrer Herstellung sind beispielsweise in der EP-A 0 311 983 oder EP-A 0 405 978 beschrieben. Außer Silicium und Titan können solche Materialien auch zusätzliche Elemente wie z. B. Aluminium, Zirkonium, Zinn, Eisen, Kobalt, Nickel, Gallium, Germanium, Bor oder geringe Menge an Fluor enthalten. In den Zeolith-Katalysatoren kann das Titan des Zeolithen teilweise oder vollständig durch Vanadium, Zirkonium, Chrom oder Niob oder einem Gemisch aus zwei oder mehr davon ersetzt sein. Das molare Verhältnis von Titan und/oder Vanadium, Zirkonium, Chrom oder Niob zur Summe aus Silicium und Titan und/oder Vanadium und/oder Zirkonium, und/oder Chrom und/oder Niob liegt in der Regel im Bereich von 0,01 : 1 bis 0,1 : 1.

Titanzeolithe, insbesondere solche mit einer Kristallstruktur vom MFI-Typ, sowie Möglichkeiten zu ihrer Herstellung sind beispielsweise in der WO 98/55228, EP-A 0 311 983 oder der EP-A 0 405 978 beschrieben, deren diesbezüglicher Umfang vollumfänglich in den Kontext der vorliegenden Anmeldung einbezogen wird.

Titanzeolithe mit MFI-Struktur sind dafür bekannt, dass sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Dabei sind im einzelnen Titan-, Germanium-, Tellur-, Vanadium-, Chrom-, Niob-, Zirkoniumhaltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen zu nennen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Bevorzugt werden im Rahmen der vorliegenden Erfindung Ti-Zeolithe mit MFI-, MEL- oder MFI/MEL-Mischstruktur eingesetzt. Als weiterhin bevorzugt sind im einzelnen die Ti-enthaltenden Zeolith-Katalysatoren, die im allgemeinen als "TS-1", "TS-2", "TS-3" bezeichnet werden, sowie Ti-Zeolithe mit einer zu beta-Zeolith isomorphen Gerüststruktur zu nennen. Ganz besonders bevorzugt ist im Rahmen der vorliegenden Erfindung ein Zeolith-Katalysator vom TS-1-Typ.

Nach der Umsetzung des Propens mit bevorzugt Wasserstoffperoxid, wobei ein Gemisch (G0) erhalten wird, wird weiter bevorzugt aus diesem Gemisch (G0), enthaltend Propylenoxid, nicht umgesetztes Propen und Methanol, nicht umgesetztes Propen abgetrennt, was bevorzugt destillativ erfolgt.

Demgemäß betrifft die vorliegende Erfindung auch ein Verfahren, wie oben beschrieben, das zusätzlich die Schritte (i) und (ii) umfasst:
(i) Umsetzung von Propen mit Wasserstoffperoxid in Anwesenheit eines Titansilikalitkatalysators und Methanol als Lösungsmittel unter Erhalt eines Gemisches (G0), enthaltend Propylenoxid, nicht umgesetztes Propen und Methanol.
(ii) Abtrennung des nicht umgesetzten Propens aus dem Gemisch (G0) unter Erhalt eines Gemisches (G1), enthaltend Propylenoxid und Methanol.

Die Umsetzung von Propen in Gegenwart von Methanol und des Titansilikalitkatalysators mittels Wasserstoffperoxid kann in einer, zwei oder auch mehr Stufen durchgeführt werden, wobei die Umsetzung besonders bevorzugt in zwei Stufen durchgeführt wird.

Eine zweistufige Umsetzung findet beispielsweise folgendermaßen statt:
(a) Umsetzung des Wasserstoffperoxids mit Propen unter Erhalt einer Mischung, umfassend Propylenoxid und nicht-umgesetztes Wasserstoffperoxid;
(b) Abtrennung des nicht-umgesetzten Wasserstoffperoxids aus der aus Stufe (a) resultierenden Mischung;
(c) Umsetzung des abgetrennten Wasserstoffperoxids aus Stufe (b) mit Propen.

Demgemäß findet die Umsetzung von Propen mit Wasserstoffperoxid, wie dargestellt, in zwei Stufen (a) und (c) statt, wobei eine Abtrennstufe (b) zwischengeschaltet ist.

Die Abtrennung des Wasserstoffperoxids in der Abtrennstufe (b), kann im Rahmen der vorliegenden Erfindung nach allen gängigen Abtrennmethoden gemäß Stand der Technik durchgeführt werden.

Vorzugsweise erfolgt die Abtrennung des Wasserstoffperoxids destillativ. Je nach den Anforderungen des Verfahrens ist dabei eine Abtrennung in einer oder mehreren Destillationskolonnen möglich. Vorzugsweise wird im Rahmen einer Abtrennstufe eine Destillationskolonne eingesetzt.

Die Umsetzung von Propen mit Wasserstoffperoxid findet im erfindungsgemäßen Verfahren in einem dafür geeigneten Reaktor statt. Als Edukte der Umsetzung werden Propen, Wasserstoffperoxid und Methanol eingesetzt. Die Edukte können im Rahmen des Verfahrens einzeln oder vorzugsweise vor dem Zufluss in den Reaktor zu einem Strom vereinigt dem Reaktor zugeführt werden. Bevorzugt wird im erfindungsgemäßen Verfahren ein Strom dem Reaktor zugeleitet, welcher aus der Kombination der Edukte besteht. Dabei ist ein Strom bevorzugt, bei dem die Konzentrationen der einzelnen Edukte des Stroms so gewählt sind, dass der Strom flüssig und einphasig ist.

In einer weiteren bevorzugten Ausführungsform ist es möglich, die Umsetzung in den Stufen (a) und (c) in zwei getrennten Reaktoren durchzuführen.

Als Reaktoren können selbstverständlich alle denkbaren, für die jeweilige Reaktion am besten geeigneten Reaktoren eingesetzt werden. Dabei ist der Begriff "ein Reaktor" nicht auf einen einzigen Behälter beschränkt. Vielmehr ist es auch möglich, als Reaktor eine Rührkesselkaskade einzusetzen.

Bevorzugt werden als Reaktoren Festbettreaktoren verwendet. Weiter bevorzugt werden als Festbettreaktoren Festbettrohrreaktoren eingesetzt, wobei auch die Suspensionsfahrweise in mindestens einem der Reaktoren möglich ist.

Insbesondere bevorzugt werden bei den Umsetzungen in den Stufen (a) und (c) in zwei getrennten Reaktoren ein isothermer Festbettrohrreaktor und ein adiabatischer Festbettreaktor eingesetzt. Bevorzugt werden der isotherme Festbettrohrreaktor in der Stufe (a) und der adiabatische Festbettreaktor in der Stufe (b) eingesetzt.

Im Rahmen einer bevorzugten Ausführungsform des erfindungsgemäßen Verfahrens enthält das Gemisch (G1) Propylenoxid in einem Anteil im Bereich von 5 bis 15 Gew.-%, bevorzugt von 6 bis 12 Gew.-% und besonders bevorzugt von 8 bis 10,5 Gew.-% und Methanol in einem Anteil im Bereich von 55 bis 85 Gew.-%, bevorzugt von 60 bis 80 Gew.-% und besonders bevorzugt von 65 bis 75 Gew.-% enthält.

Der Begriff "ein Brüdenstrom, im wesentlichen enthaltend Propylenoxid", wie er im Rahmen der vorliegenden Anmeldung bezüglich des Brüdenstroms verwendet wird, der aus Schritt (iii) erhalten wird, bezeichnet einen Brüdenstrom, der einen Lösungsmittelgehalt von höchstens 500 ppm, bevorzugt von höchstens 200 ppm, weiter bevorzugt von höchstens 100 ppm, weiter bevorzugt von höchstens 50 ppm, weiter bevorzugt von höchstens 20 ppm, besonders bevorzugt von höchstens 15 ppm und insbesondere bevorzugt von höchstens 10 ppm, jeweils bezogen auf das Gesamtgewicht der abgetrennten Propylenoxidfraktion, aufweist.

Gegenüber dem aus dem Stand der Technik bekannten Verfahren weist das erfindungsgemäße Verfahren unter anderem den Vorteil auf, dass es mit niedrigeren Rücklaufverhältnissen betrieben werden kann. Erfindungsgemäß liegen diese Rücklaufverhältnisse bevorzugt im Bereich von 4 bis 10. Unter anderem bevorzugte Rücklaufverhältnisse sind etwa 4, 5, 6, 7, 8, 9 oder 10.

Die vorliegende Erfindung wird anhand der nachfolgenden Beispiele näher erläutert.

### Beispiele

### Vergleichsbeispiel 1: Herkömmliche Auftrennung unter Erhalt einer Propylenoxidfraktion mit einem Methanolgehalt von 20 ppm

Gemäß dem in der WO 00/07965 angegebenen Verfahren wird Propylenoxid ausgehend von Propen durch Umsetzung mit Wasserstoffperoxid in Methanol als Lösungsmittel unter Verwendung eines Titanzeolithkatalysators vom Typ TS-1 hergestellt. Aus dem nach der Abtrennung des überschüssigen Propens entstehenden Gemisch mit der unten aufgeführten Zusammensetzung wird Propylenoxid derart destillativ abgetrennt, dass die resultierende Propylenoxidfraktion eine Methanolkonzentration von 20 ppm aufweist.

Zusammensetzung des zu trennenden Gemischs:

| | |
|---|---|
| Leichtsieder: | 0,2 Gew.-% |
| Propylenoxid: | 9,3 Gew.-% |
| Methanol: | 70 Gew.-% |
| Methoxypropanole: | 0,5 Gew.-% |
| Wasser: | 18 Gew.-% |
| Hochsieder: | 2 Gew.-% |

Die Destillation wird bei einem Druck von 1,5 bar durchgeführt, so dass der Brüden im Kondensator mit zugänglichem Flusswasser bei einer Temperatur von etwa 42 °C kondensiert werden kann. Die Methanolkonzentration des Propylenoxidstromes beträgt 20 ppm. Die optimierte Trennung verläuft bei einem Rücklaufverhältnis von 18 und weist einen Energiebedarf für die obige Trennung von 2.650 kWh/t (Propylenoxidfraktion) auf.

### Vergleichsbeispiel 2: Herkömmliche Auftrennung unter Erhalt einer Propylenoxidfraktion mit einem Methanolgehalt von 10 ppm

Gemäß dem in der WO 00/07965 angegebenen Verfahren wird Propylenoxid ausgehend von Propen durch Umsetzung mit Wasserstoffperoxid in Methanol als Lösungsmittel unter Verwendung eines Titanzeolithkatalysators vom Typ TS-1 hergestellt. Aus dem nach der Abtrennung des überschüssigen Propens entstehenden Gemisch mit der unten aufgeführten Zusammensetzung wird Propylenoxid derart destillativ abgetrennt, dass die resultierende Propylenoxidfraktion eine Methanolkonzentration von 10 ppm aufweist.

Zusammensetzung des zu trennenden Gemischs:

| | |
|---|---|
| Leichtsieder: | 0,2 Gew.-% |
| Propylenoxid: | 9,3 Gew.-% |
| Methanol: | 70 Gew.-% |
| Methoxypropanole: | 0,5 Gew.-% |
| Wasser: | 18 Gew.-% |
| Hochsieder: | 2 Gew.-% |

Die Destillation wird bei einem Druck von 1,5 bar durchgeführt, so dass der Brüden im Kondensator mit zugänglichem Flusswasser bei einer Temperatur von etwa 42 °C kondensiert werden kann. Die Methanolkonzentration des Propylenoxidstromes beträgt 10 ppm. Die optimierte Trennung verläuft bei einem Rücklaufverhältnis von 23 und weist einen Energiebedarf für die obige Trennung von 3.280 kWh/t (Propylenoxidfraktion) auf.

### Beispiel 1 Erfindungsgemäße Auftrennung unter Erhalt einer Propylenoxidfraktion mit einem Methanolgehalt von 20 ppm

Gemäß dem in der WO 00/07965 angegebenen Verfahren wird Propylenoxid ausgehend von Propen durch Umsetzung mit Wasserstoffperoxid in Methanol als Lösungsmittel unter Verwendung eines Titanzeolithkatalysators vom Typ TS-1 hergestellt. Aus dem nach der Abtrennung des überschüssigen Propens entstehenden Gemisch mit der unten aufgeführten Zusammensetzung wird Propylenoxid derart destillativ abgetrennt, dass die resultierende Propylenoxidfraktion eine Methanolkonzentration von 20 ppm aufweist.

Zusammensetzung des zu trennenden Gemischs:

| | |
|---|---|
| Leichtsieder: | 0,2 Gew.-% |
| Propylenoxid: | 9,3 Gew.-% |
| Methanol: | 70 Gew.-% |
| Methoxypropanole: | 0,5 Gew.-% |
| Wasser: | 18 Gew.-% |
| Hochsieder: | 2 Gew.-% |

Die Destillation wird bei einem Druck von 500 mbar unter Verwendung eines Brüdenverdichters und Einsatz des verdichteten Brüden zum Betrieb des Naturumlaufverdampfers der Kolonne durchgeführt. Der Brüden wird auf einen Druck von etwa 2,8 bar mit Hilfe eines Turboverdichters (einstufiger Wellenverdichter mit 2 x 4 Laufrädern) verdichtet, wobei sich im verdichteten Brüden eine Temperatur von etwa 68 °C einstellt. Der Bedarf des Verdichters an elektrischer Energie beträgt etwa 6 MW. Die Verdampfungstemperatur im Umlaufverdampfer der Kolonne liegt bei etwa 54 °C.

Das Rücklaufverhältnis bei dieser Trennung (Methanolrestkonzentration von 20 ppm im Propylenoxidstrom) beträgt etwa 8. Dabei ergibt sich neben der Wärmekopplung ein zusätzlicher Energiebedarf für den Antrieb des Verdichters von etwa 180 kWh/t (Propylenoxidfraktion).

### Beispiel 2 Erfindungsgemäße Auftrennung unter Erhalt einer Propylenoxidfraktion mit einem Methanolgehalt von 10 ppm

Gemäß dem in der WO 00/07965 angegebenen Verfahren wird Propylenoxid ausgehend von Propen durch Umsetzung mit Wasserstoffperoxid in Methanol als Lösungsmittel unter Verwendung eines Titanzeolithkatalysators vom Typ TS-1 hergestellt. Aus dem nach der Abtrennung des überschüssigen Propens entstehenden Gemisch mit der unten aufgeführten Zusammensetzung wird Propylenoxid derart destillativ abgetrennt, dass die resultierende Propylenoxidfraktion eine Methanolkonzentration von 10 ppm aufweist.

Zusammensetzung des zu trennenden Gemischs:

| | |
|---|---|
| Leichtsieder: | 0,2 Gew.-% |
| Propylenoxid: | 9,3 Gew.-% |
| Methanol: | 70 Gew.-% |
| Methoxypropanole: | 0,5 Gew.-% |
| Wasser: | 18 Gew.-% |
| Hochsieder: | 2 Gew.-% |

Die Destillation wird bei einem Druck von 500 mbar unter Verwendung eines Brüdenverdichters und Einsatz des verdichteten Brüden zum Betrieb des Naturumlaufverdampfers der Kolonne durchgeführt. Der Brüden wird auf einen Druck von etwa 2,8 bar mit Hilfe eines Turboverdichters (einstufiger Wellenverdichter mit 2 x 4 Laufrädern) verdichtet, wobei sich im verdichteten Brüden eine Temperatur von etwa 68 °C einstellt. Der Bedarf des Verdichters an elektrischer Energie beträgt etwa 8 MW. Die Verdampfungstemperatur im Umlaufverdampfer der Kolonne liegt bei etwa 54 °C.

Das Rücklaufverhältnis bei dieser Trennung (Methanolrestkonzentration von 10 ppm im Propylenoxidstrom) beträgt etwa 9. Dabei ergibt sich neben der Wärmekopplung ein zusätzlicher Energiebedarf für den Antrieb des Verdichters von etwa 200 kWh/t (Propylenoxidfraktion).

## Patentansprüche

1. Verfahren zur Herstellung von Propylenoxid, mindestens umfassend die Schritte (iii) und (iv)
(iii) Destillative Abtrennung von Propylenoxid aus einem Gemisch (G1), enthaltend Propylenoxid und mindestens ein Lösungsmittel, unter Verwendung einer Destillationskolonne, wobei ein Sumpfstrom und ein Brüdenstrom, im Wesentlichen enthaltend Propylenoxid, erhalten wird;
(iv) Komprimierung des gemäß (iii) erhaltenen Brüdenstroms mittels mindestens eines Verdichters unter Erhalt eines verdichteten Brüden.

2. Verfahren nach Anspruch 1, **dadurch gekennzeichnet, dass** das mindestens eine Lösungsmittel Methanol ist.

3. Verfahren nach Anspruch 1 oder 2, **dadurch gekennzeichnet, dass** die zur destillativen Abtrennung gemäß (iii) verwendete Destillationskolonne bei einem Druck im Bereich von 450 bis 750 mbar betrieben wird.

4. Verfahren nach einem der Ansprüche 1 bis 3, **dadurch gekennzeichnet, dass** die Verdichtung des Brüden mit einem Turboverdichter erfolgt.

5. Verfahren nach einem der Ansprüche 1 bis 4, **dadurch gekennzeichnet ist, dass** der Brüden gemäß (iv) auf einen Druck im Bereich von 2 bis 5 bar komprimiert wird und der komprimierte Brüden eine Temperatur aufweist, die in einem Bereich von 8 bis 20 °C über der Temperatur des in der Destillationskolonne gemäß (iii) verdampfenden Mediums liegt.

6. Verfahren nach einem der Ansprüche 1 bis 5, zusätzlich umfassend den folgenden Schritt (v)
(v) Kondensation des gemäß (iv) erhaltenen Brüden unter Rückführung mindestens einen Teils der Kondensationswärme in mindestens einen in der gemäß (iii) verwendeten Destillationskolonne eingesetzten Verdampfer.

7. Verfahren nach Anspruch 6, zusätzlich umfassend folgenden Schritt (vi)
(vi) Abkühlen mindestens eines Teils des gemäß (v) erhaltenen Kondensats in mindestens einem Wärmetauscher auf eine Temperatur im Bereich von 10 bis 30 °C und Rückführung dieses Teils des abgekühlten Kondensats als Rücklauf auf die gemäß (iii) verwendete Destillationskolonne.

8. Verfahren nach Anspruch 7, **dadurch gekennzeichnet, dass** in dem mindestens einen gemäß (vi) eingesetzten Wärmetauscher komprimiertes Propen unter Entspannung vollständig verdampft wird.

9. Verfahren nach einem der Ansprüche 1 bis 8, **dadurch gekennzeichnet, dass** die im gemäß (iii) erhaltenen Sumpfstrom gespeicherte Energie zumindest teilweise zur Aufheizung des Gemisches (G1) vor dessen destillativer Auftrennung gemäß (iii) eingesetzt wird.

10. Verfahren nach einem der Ansprüche 1 bis 9, zusätzlich umfassend die Schritte (i) und (ii)
(i) Umsetzung von Propen mit Wasserstoffperoxid in Anwesenheit eines Titansilikalitkatalysators und Methanol als Lösungsmittel unter Erhalt eines Gemisches (G0), enthaltend Propylenoxid, nicht umgesetztes Propen und Methanol.
(ii) Abtrennung des nicht umgesetzten Propens aus dem Gemisch (G0) unter Erhalt eines Gemisches (G1), enthaltend Propylenoxid und Methanol.

## Claims

1. A process for preparing propylene oxide, which comprises at least the steps (iii) and (iv):
(iii) separating off propylene oxide from a mixture (M1) comprising propylene oxide and at least one solvent by distillation in a distillation column, giving a bottom stream and a vapor stream consisting essentially of propylene oxide;
(iv) compressing the vapor stream obtained in (iii) by means of at least one compressor to give a compressed vapor.

2. The process according to claim 1, wherein the at least one solvent is methanol.

3. The process according to claim 1 or 2, wherein the distillation column used for the separation by distillation in (iii) is operated at a pressure in the range from 450 to 750 mbar.

4. The process according to any of claims 1 to 3, wherein the compression of the vapor is carried out using a turbocompressor.

5. The process according to any of claims 1 to 4, wherein the vapor is compressed to a pressure in the range from 2 to 5 bar in (iv) and the compressed vapor has a temperature which is from 8 to 20°C above the temperature of the medium vaporizing in the distillation column in (iii).

6. The process according to any of claims 1 to 5, which additionally comprises the step (v):
(v) condensing the vapor obtained in (iv) and returning at least part of the heat of condensation to at least one vaporizer used in the distillation column employed in (iii).

7. The process according to claim 6, which additionally comprises the step (vi):
(vi) cooling at least part of the condensate obtained in (v) to a temperature in the range from 10 to 30°C in at least one heat exchanger and returning this part of the cooled condensate as runback to the distillation column used in (iii).

8. The process according to claim 7, wherein propene compressed in the heat exchanger(s) used in (vi) is completely vaporized with depressurization.

9. The process according to any of claims 1 to 8, wherein the energy stored in the bottom stream obtained in (iii) is at least partly used for heating the mixture (M1) before it is fractionally distilled in (iii).

10. The process according to any of claims 1 to 9, which additionally comprises the steps (i) and (ii) :
(i) reacting propene with hydrogen peroxide in the presence of a titanium silicalite catalyst and methanol as solvent to give a mixture (M0) comprising propylene oxide, unreacted propene and methanol;
(ii) separating off the unreacted propene from the mixture (M0) to give a mixture (M1) comprising propylene oxide and methanol.

## Revendications

1. Procédé de préparation d'oxyde de propylène, comprenant au moins les étapes (iii) et (iv) consistant à :
(iii) isoler de l'oxyde de propylène par distillation à partir d'un mélange (G1), contenant de l'oxyde de propylène et au moins un solvant, avec mise en oeuvre d'une colonne de distillation, un courant de pied et un courant de buées, contenant essentiellement de l'oxyde de propylène, étant obtenu ;
(iv) comprimer le courant de buées obtenu en (iii) par l'intermédiaire d'au moins un compresseur, avec obtention de buées comprimées.

2. Procédé selon la revendication 1, **caractérisé en ce que** le au moins un solvant est du méthanol.

3. Procédé selon la revendication 1 ou 2, **caractérisé en ce que** la colonne de distillation mise en oeuvre en (iii) pour l'isolation par distillation fonctionne à une pression dans la plage de 450 à 750 mbars.

4. Procédé selon l'une quelconque des revendications 1 à 3, **caractérisé en ce que** la compression des buées a lieu avec un turbocompresseur.

5. Procédé selon l'une quelconque des revendications 1 à 4, **caractérisé en ce que** les buées sont comprimées en (iv) à une pression dans la plage de 2 à 5 bars et les buées comprimées présentent une température qui se situe dans une plage de 8 à 20 °C au-dessus de la température du milieu s'évaporant en (iii) dans la colonne de distillation.

6. Procédé selon l'une quelconque des revendications 1 à 5, comprenant de manière supplémentaire l'étape (v) ci-dessous consistant à :
(v) condenser les buées obtenues en (iv) avec recyclage d'au moins une partie de la chaleur de condensation dans au moins un évaporateur utilisé dans la colonne de distillation mise en oeuvre en (iii).

7. Procédé selon la revendication 6, comprenant de manière supplémentaire l'étape (vi) ci-dessous consistant à :
(vi) refroidir au moins une partie du condensat obtenu en (v) dans au moins un échangeur de chaleur à une température dans la plage de 10 à 30 °C et recycler cette partie du condensat refroidi en tant que reflux vers la colonne de distillation mise en oeuvre en (iii).

8. Procédé selon la revendication 7, **caractérisé en ce que** du propène comprimé est évaporé intégralement avec détente, dans le au moins un échangeur de chaleur utilisé en (vi).

9. Procédé selon l'une quelconque des revendications 1 à 8, **caractérisé en ce que** l'énergie accumulée dans le courant de pied obtenu en (iii) est utilisée en (iii) au moins partiellement pour le chauffage du mélange (G1) avant sa séparation par distillation.

10. Procédé selon l'une quelconque des revendications 1 à 9, comprenant de manière supplémentaire les étapes (i) et (ii) consistant à :
(i) faire réagir du propène avec du peroxyde d'hydrogène en présence d'un catalyseur à la silicalite de titane et de méthanol en tant que solvant, avec obtention d'un mélange (G0) contenant de l'oxyde de propylène, du propène non réagi et du méthanol.
(ii) isoler le propène non réagi à partir du mélange (G0), avec obtention d'un mélange (G1) contenant de l'oxyde de propylène et du méthanol.
